# EUROPÄISCHE PATENTSCHRIFT

(11) **EP 2 449 119 B1**
(45) Veröffentlichungstag und Bekanntmachung des Hinweises auf die Patenterteilung: **17.04.2013**
(21) Anmeldenummer: 10737797.0
(22) Anmeldetag: 29.06.2010
(51) Int. Cl.: C12P 7/06, B01D 1/00, B01D 1/14, B01D 3/00, B01D 1/06, B01D 1/26, B01D 3/14, C13B 5/00, C13B 10/00, C13B 10/12

(54) **ANLAGE ZUR HERSTELLUNG VON ALKOHOL AUS ZUCKERROHR**
INSTALLATION FOR PRODUCING ETHANOL FROM SUGAR CANE
INSTALLATION DE PRODUCTION D'ALCOOL À PARTIR DE CANNE À SUCRE

(30) Priorität: 29.06.2009 DE 102009030960
(43) Veröffentlichungstag der Anmeldung: 09.05.2012
(73) Patentinhaber: GEA Wiegand GmbH, 76275 Ettlingen (DE)
(72) Erfinder: SCHORMÜLLER, Matthias, 76185 Karlsruhe (DE)
(74) Vertreter: Liska, Horst
(86) Internationale Anmeldenummer: PCT/EP2010/059210
(87) Internationale Veröffentlichungsnummer: WO 2011/000831

(56) Entgegenhaltungen:
- EP-A2- 0 048 061
- WO-A1-2004/038031
- RAVAGNANI M A S S ET AL: "Improving Energetic Performance and Water Usage in an Industrial Ethanol Distillery", PROCESS SAFETY AND ENVIRONMENTAL PROTECTION, INSTITUTION OF CHEMICAL ENGINEERS, RUGBY, GB, Bd. 85, Nr. 6, 1. Januar 2007 (2007-01-01) , Seiten 526-532, XP022618615, ISSN: 0957-5820, DOI: DOI:10.1205/PSEP06059 [gefunden am 2007-01-01]

## Beschreibung

Die Erfindung betrifft eine Anlage zur Herstellung von Alkohol (Ethanol) aus Zuckerrohr und insbesondere eine Anlage zur industriellen Herstellung von Alkohol.

Es ist bekannt, Alkohol (Ethanol) aus nachwachsenden Rohstoffen, insbesondere auch aus Zuckerrohr in industriellem Ausmaß, zum Beispiel als Treibstoff für Kraftfahrzeuge herzustellen. Das Zuckerrohr wird hierbei zunächst zerkleinert, zum Beispiel gequetscht und zerhäckselt. Aus dem zerkleinerten Zuckerrohr wird dann beispielsweise durch Auspressen der Zuckersaft extrahiert. Zusätzlich kann durch Auswaschen mit heißem Wasser der Extraktionsgrad erhöht werden. Der Zuckersaft wird sodann gereinigt, beispielsweise gefiltert, wobei vor dem Filtern durch Zusatz von gebranntem Kalk und Erwärmen des Zuckersaftes ein Teil der unlöslichen Bestandteile des Zuckersaftes ausgefällt werden können.

Nach dieser Vorbehandlung des Zuckersaftes wird der Zuckersaft, wie in der Alkoholherstellung üblich, vergoren. Aus der beim Gärprozess entstehenden, alkoholhaltigen Maische wird in einem Destillationsprozess und gegebenenfalls in einem nachfolgenden Rektifikationsprozess der Alkoholanteil der Maische abdestilliert. Da speziell für Treibstoffanwendungen dehydrierter Alkohol erwünscht ist, folgt in aller Regel eine Entwässerung zur Erzeugung von dehydriertem Alkohol.

Der Alkoholherstellungsprozess ist energieintensiv. In einer Vielzahl der Prozessstufen muss Prozessenergie zugeführt werden. Insbesondere haben die Destillation und Entwässerung des Alkohols vergleichsweise hohen Energiebedarf. Andererseits fallen bei der Herstellung von Alkohol aus Zuckerrohr Restprodukte an, die zur Deckung des Energiebedarfs bei der Herstellung des Alkohols, aber auch zur Deckung der Herstellungskosten mit herangezogen werden können.

Bei der Extrahierung des Zuckersaftes aus dem Zuckerrohr fällt Bagasse, d.h. der Faseranteil des Zuckerrohrs an. Es ist bekannt, die Bagasse zum Beheizen eines Hochdruckdampferzeugers zu verbrennen und mit dem so erzeugten Hochdruckdampf im Sinne einer Wärme-Kraft-Kopplung eine Gegendruckturbine anzutreiben, die ihrerseits einen elektrische Energie erzeugenden Generator treibt. Es ist ferner bekannt, mit dem Abdampf der Gegendruckturbine die für die Reinigung des Zuckersaftes, die Destillation der Maische, die Rektifikation des Rohalkohols und die Alkoholentwässerung erforderlichen Apparate zu beheizen. Die von dem Generator erzeugte elektrische Energie kann in der Alkoholherstellungsanlage genutzt werden, wird aber vielfach in öffentliche Stromnetze eingespart, um mit dem hierfür eingenommenen Gebühren die Kosten der Alkoholherstellung zu "subventionieren".

Für den vorstehend erläuterten Alkoholherstellungsprozess wird typischerweise 4,5 kg Dampf pro Liter Alkohol benötigt. Diese Dampfmenge lässt sich durch das Verbrennen der Bagasse aus dem Abdampf der den elektrischen Generator treibenden Gegendruckturbine gewinnen, sodass sich unter Berücksichtigung der zusätzlich erzeugten elektrischen Energie der Alkoholherstellungsprozess hinreichend wirtschaftlich betreiben lässt.

Bei der Destillation und Rektifikation fällt als Restprodukt sirupartige Vinasse an, die zum überwiegenden Teil aus organischen Substanzen besteht und als Düngemittel, ggf. auch als Tierfutter genutzt werden kann. In der bei der Destillation und Rektifikation anfallenden Form hat Vinasse einen relativ hohen Flüssigkeitsanteil und dementsprechend entstehen hohe Transportkosten, wenn die Vinasse in dieser Form als Düngemittel ausgebracht werden soll.

Es ist bekannt, die Vinasse vor dem Transport einzudampfen bzw. zu konzentrieren. Hierzu werden mehrstufige Eindampfanlagen eingesetzt, die aber zusätzliche Energie benötigen, welche bei herkömmlichen Alkoholherstellungsprozessen nicht aus dem Prozess selbst bereitgestellt werden kann.

Es ist Aufgabe der Erfindung, eine Anlage zur industriellen Herstellung von Alkohol (Ethanol) aus Zuckerrohr zu schaffen, die es nicht nur erlaubt, überschüssige elektrische Energie aus dem Herstellungsprozess abzuziehen, sondern auch die in dem Herstellungsprozess anfallende Vinasse zu konzentrieren.

Die Erfindung geht aus von einer Anlage zur Herstellung aus Alkohol (Ethanol) aus Zuckerrohr, umfassend
- eine das Zuckerrohr zerkleinernde Zerkleinerungsstufe,
- eine das zerkleinerte Zuckerrohr in Zuckersaft und Bagasse trennende Extraktionsstufe,
- eine den Zuckersaft reinigende Reinigungsstufe,
- eine den gereinigten Zuckersaft zu alkoholhaltiger Maische vergärende Gärstufe,
- eine die Maische in Rohalkohol und Vinasse trennende Destillationsanlage,
- eine den Rohalkohol entwässernde, dehydrierten Alkohol liefernde Entwässerungsstufe,
- einen durch Verbrennen der Bagasse beheizten Hochdruckdampferzeuger und
- eine mit dem Hochdruckdampf des Hochdruckdampferzeugers betriebene Dampfturbine, die einen elektrischen Strom erzeugenden Generator antreibt.

Die erfindungsgemäße Verbesserung ist dadurch gekennzeichnet, dass der gereinigte Zuckersaft der Gärstufe über eine mit Abdampf der Dampfturbine beheizte Vorkonzentrierungsstufe in aufkonzentrierter Form zuführbar ist, und dass die Destillationsanlage sowie die Entwässerungsstufe und eine die Vinasse der Destillationsanlage aufkonzentrierende Verdampferanordnung gemeinsam mit Abdampf der Vorkonzentrierungsstufe beheizt sind; insbesondere direkt, d.h. unmittelbar beheizt sind.

Da der gereinigte Zuckersaft vor der Vergärung konzentriert wird, arbeitet der Destillationsprozess mit vorkonzentrierter, alkoholhaltiger Maische und kann deshalb mit verringertem Energiebedarf betrieben werden. Die Energie für die Vorkonzentrierung des gereinigten Zuckersaftes wird durch den Abdampf der Dampfturbine bereitgestellt. Der Abdampf der Vorkonzentrierungsstufe wird in drei Teile aufgeteilt und liefert die Energie für den Destillationsprozess, den Entwässerungsprozess und zusätzlich die für die Aufkonzentrierung der Vinasse benötigte Energie. Es hat sich gezeigt, dass sich die Vinasse auf ein Zehntel ihres Volumens eindampfen lässt, obwohl der gesamte Alkoholherstellungsprozess nach wie vor nur etwa 4,5 kg Dampf Liter Alkohol benötigt, wie dies vorangegangen als typischerweise erforderlichen Energiebedarf angegeben wurde.

In herkömmlichen Alkoholherstellungsanlagen der genannten Art hat der Abdampf der Dampfturbine beispielsweise eine Kondensationstemperatur von 127° C (entsprechend 2,5 bar, a (2,5·10⁵P a)). Bei der Vorkonzentrierung des gereinigten Zuckersaftes fällt Abdampf bei einer Kondensationstemperatur von beispielsweise 120° C (entsprechend 2 bar, a (2·10⁵ Pa)) an. Damit ein erster Teil des Abdampfs der Vorkonzentrierungsstufe für die Destillation/Rektifikation ausreicht, ist in einer bevorzugten Ausgestaltung vorgesehen, dass die Destillationsanlage zweistufig ausgebildet ist und eine mit Abdampf der Vorkonzentrierungsstufe direkt beheizte erste Destillationskolonne, sowie eine indirekt durch Kopfbrüden der ersten Destillationskolonne beheizte zweite Destillationskolonne aufweist, die auf einem niedrigeren Kolonnendruck und einer niedrigeren Kolonnentemperatur arbeitet als die erste Destillationskolonne. Zweckmäßigerweise arbeitet die erste Destillationskolonne als "Druck-Kolonne" bei einem Druck von 1,6 +/- 0,5 bar, a (1,6·10⁵ +/- 0,5·10⁵ Pa) in ihrem Kopfbereich und/oder einer Temperatur von etwa 90° C (bei 1,6 bar, a (1,6·10⁵ Pa)), während die zweite Destillationskolonne als "Vakuum-Kolonne" arbeitet und in ihrem Sumpfbereich vorzugsweise bei einem Druck von 0,5 +/- 0,3 bar, a (0,5·10⁵ +/- 0,3·10⁵ Pa) und/oder einer Temperatur von etwa 80° C betrieben wird. Zweckmäßigerweise sind die beiden Destillationskolonnen maischezuführseitig und gegebenenfalls auch vinasseausgangsseitig parallel geschaltet. Den beiden Destillationskolonnen sind gesonderte Kopfbrüdenkondensatoren zugeordnet, wobei der Kopfbrüdenkondensator der ersten Destillationskolonne einen Reboiler der zweiten Destillationskolonne bildet. Die in dem Reboiler zirkulierende Vinasse der zweiten, kälteren Destillationskolonne kondensiert den Kopfbrüdenalkohol der ersten Destillationskolonne. Ein Teil des Kondensats wird der ersten Destillationskolonne als Rücklauf wieder zugeführt. Der Kopfbrüdenkondensator der zweiten Destillationskolonne wird mit Kühlwasser gekühlt. Und auch hier wird ein Teil des Kondensats der zweiten Destillationskolonne als Rücklauf wieder zugeführt.

Während der zweite Teil des Abdampfes der Vorkonzentrierungsstufe die für den Betrieb der Entwässerungsstufe, beispielsweise eines Molekularsiebs oder dergleichen, benötigte Energie liefert, wird der dritte Teil des Abdampfes der Vorkonzentrierungsstufe für den Betrieb der die Vinasse aufkonzentrierenden Verdampferanordnung ausgenutzt. Die Verdampferanordnung ist hierzu mehrstufig, zweckmäßigerweise sechsstufig ausgebildet, wobei der größte Teil der Stufen bevorzugt als Fallstromverdampfer ausgeführt sind.

Die Verdampferstufen sind untereinander vinasseseitig in Reihe geschaltet und werden jeweils durch Abdampf einer in der Reihe vorangehenden, auf* höherem Druck- oder Temperaturniveau sich befindenden Verdampferstufe beheizt, sodass die in der Reihe erste Verdampferstufe bei der höchsten Temperatur und dem höchsten Druck arbeitet, während Druck und Temperatur der letzten Verdampferstufe am niedrigsten sind. Die Vinassekonzentration nimmt zur letzten Verdampferstufe hin zu. Die Reihenfolge, mit der die Verdampferstufen vinasseseitig aufeinander folgen kann von der Reihenfolge, in der sie mit Bezug auf den Abdampf aufeinander folgen abweichen. Auf diese Weise kann den sich ändernden Viskositätseigenschaften der stufenweise mehr und mehr aufkonzentrierten Vinasse besser Rechnung getragen werden. Folgen beispielsweise die Verdampferstufen abdampfseitig in der Reihenfolge erste, zweite, dritte, vierte, fünfte und sechste Stufe aufeinander, so hat sich eine vinasseseitige Reihenfolge als zweckmäßig erwiesen, in der die erste, die zweite, die dritte, die sechste, die fünfte und die vierte Verdampferstufe aufeinander folgen.

In einer bevorzugten Ausgestaltung ist eine die Vinasse der ersten Destillationskolonne aufnehmende erste Verdampferstufe der Verdampferanordnung durch Abdampf der Vorkonzentrierungsstufe direkt beheizt. Die Vinasse der zweiten Destillationskolonne kann gleichfalls der ersten Verdampferstufe zugeführt werden, ist aber zur besseren Energieausnutzung bevorzugt einer weiteren der Verdampferstufen zuführbar, beispielsweise der dritten Verdampferstufe.

Die Vorkonzentrierungsstufe ist bevorzugt als Fallfilmverdampfer ausgebildet; es kann sich aber auch um einen Plattenverdampfer handeln. Es hat sich als zweckmäßig erwiesen, die Vorkonzentrierungsstufe als einstufigen Verdampfer auszubilden. Der Abdampf der Vorkonzentrierungsstufe sollte bevorzugt einen Druck von 2 +/- 0,5 bar, a (2·10⁵ +/- 0,5·10⁵ Pa) und/oder eine Temperatur von etwa 120° C haben.

Im Folgenden wir ein Ausführungsbeispiel der Erfindung anhand einer Zeichnung näher erläutert. Hierbei zeigt:
Fig. 1 eine schematische Darstellung einer erfindungsgemäßen Anlage zur industriellen Herstellung von Alkohol aus Zuckerrohr;
Fig. 2 eine schematische Darstellung einer Destillationsanlage der Alkoholherstellungsanlage und
Fig. 3 eine schematische Darstellung einer die Vinasse der Destillationsanlage aufkonzentrierenden Verdampferanordnung der Alkoholherstellungsanlage.

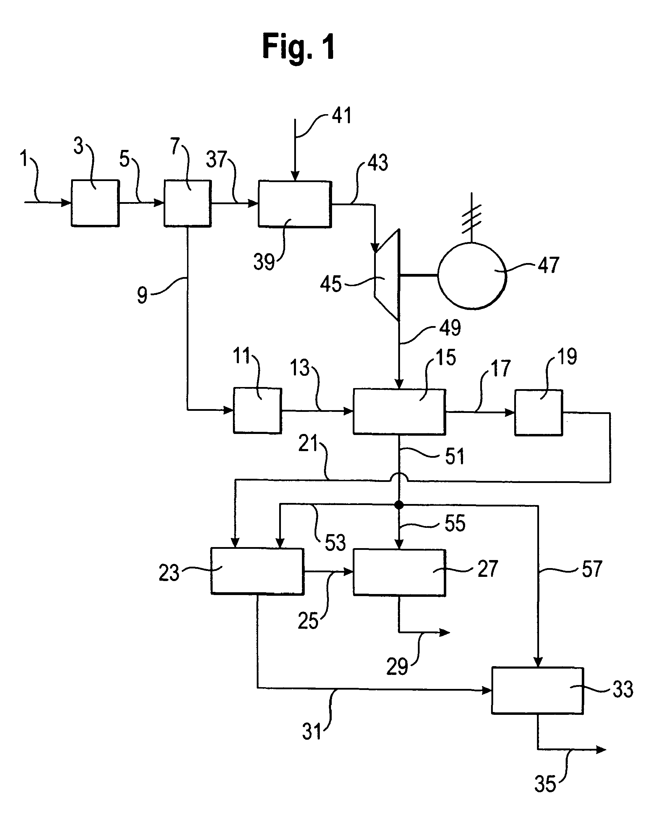

Fig. 1 zeigt schematisch eine Anlage zur industriellen Herstellung von Alkohol (Ethanol) aus Zuckerrohr, die es erlaubt, neben der Herstellung von dehydriertem Alkohol noch einen Überschuss an elektrischer Energie zu erzeugen, und bei der Destillation anfallende Vinasse aufzukonzentrieren.

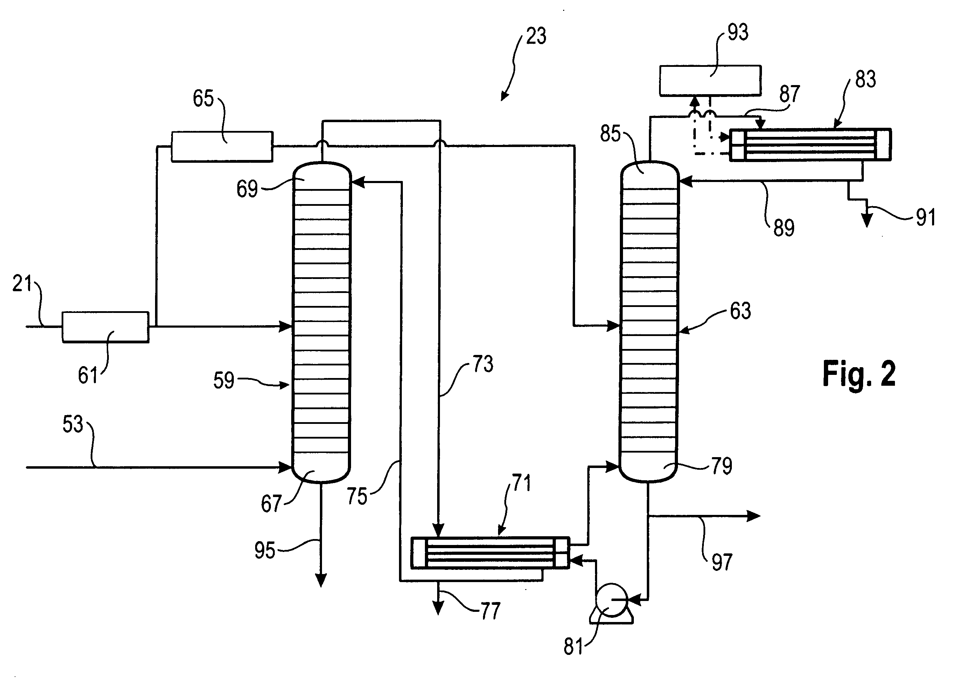

Das zu Alkohol zu verarbeitende Zuckerrohr wird bei 1 einer Zerkleinerungsstufe 3 zugeführt, die das Zuckerrohr in Stücke von beispielsweise 10 bis 20 cm Länge zerhäckselt und/oder zerquetscht. Das zerkleinerte Zuckerrohr wird bei 5 einer Extraktionsstufe 7 zugeführt, die aus dem zerkleinerten Zuckerrohr den Zuckersaft extrahiert, beispielsweise auspresst und/oder durch heißes Wasser auswäscht. Der bei 9 austretende Zuckersaft wird in einer Reinigungsstufe 11 von für die Destillation unerwünschten Stoffen gereinigt. Beispielsweise filtert die Reinigungsstufe 11 Faserstoffe oder Sand oder dergleichen aus dem Zuckersaft. Darüber hinaus kann in der Reinigungsstufe 11 gebrannter Kalk (CaO) zum Ausfällen und Ausflocken unlöslicher Bestandteile des Zuckersaftes zugesetzt werden, wie dies bei der Zuckerherstellung üblich ist. Der gereinigte Zuckersaft wird bei 13 einer nachfolgend noch näher erläuterten Vorkonzentrierungsstufe 15 zugeführt, die den Zuckersaft aufkonzentriert bzw. in einem gewissen Umfang eindampft, bevor der aufkonzentrierte Zuckersaft bei 17 einer Gärstufe 19 zugeführt wird, in welcher der aufkonzentrierte Zuckersaft in üblicher Weise zu alkoholhaltiger Maische vergoren wird. Die alkoholhaltige Maische wird bei 21 einer anhand von Fig. 2 nachfolgend noch näher erläuterten Destillationsanlage 23 zugeführt, die bei 25 rektifizierten Alkohol an eine Entwässerungsstufe 27 abgibt. Die Entwässerungsstufe 27 liefert an ihrem Produktausgang 29 dehydrierten, rektifizierten Alkohol. In der Destillationsanlage 23 im Destillations-/Rektifikationsprozess als Restprodukt anfallende Vinasse wird bei 31 einer Verdampferanordnung 33 zugeführt, die den anfänglich vergleichsweise hohen Wasseranteil der Vinasse verringert und die beispielsweise auf ein Zehntel des ursprünglichen Vinassevolumens aufkonzentrierte Vinasse an einem Produktausgang 35 abgibt.

In der Extraktionsstufe 7 fällt neben dem Zuckersaft als Restprodukt Bagasse an. Bei Bagasse handelt es sich um den Feststoffanteil des Zuckerrohrs. Die Bagasse wird bei 37 einem Hochdruckdampferzeuger 39 zugeführt, der durch Verbrennen der Bagasse aus bei 41 zugeführtem Frischwasser Hochdruckdampf erzeugt. Der Hochdruckdampf speist über eine Leitung 43 eine Gegendruckturbine 45, die ihrerseits einen elektrischen Strom erzeugenden Generator 47 antreibt. Bei der Gegendruckturbine 45 handelt es sich um eine Dampfturbine, bei der Abdampf nicht bis in den Unterdruck-Bereich hinein entspannt wird, sondern bei 49 im ÜberdruckBereich und überhitzt entnommen wird. Beispielsweise hat der Turbine abdampf eine Kondensationstemperatur von 127° C (entsprechend 2,5 bar, a (2,5·10⁵ Pa)).

Der Abdampf der Gegendruckturbine 45 beheizt die Vorkonzentrierungsstufe 15, bei der es sich um einen einstufigen Fallfilmverdampfer oder einen einstufigen Plattenverdampfer handeln kann. Da die Vorkonzentrierungsstufe 15 den gereinigten Zuckersaft aufkonzentriert, bevor der Zuckersaft in der Gärstufe 19 vergoren wird, wird auch die Destillationsanlage 23 mit aufkonzentrierter Maische beschickt, was den Energiebedarf der Destillationsanlage 23 mindert.

Der bei 51 die Vorkonzentrierungsstufe 15 verlassende Abdampf hat beispielsweise eine Kondensationstemperatur von 120° C, entsprechend 2 bar, a(2·10⁵ Pa). Dieser Abdampf wird auf drei Teile aufgeteilt, von denen ein erster Teil bei 53 der Destillationsanlage 23, ein zweiter Teil bei 55 der Entwässerungsstufe 27 und ein dritter Teil bei 57 der Verdampferanordnung 33 jeweils zur Beheizung und Energiezufuhr zugeführt wird. Die erfindungsgemäße Alkoholherstellungsanlage erzeugt damit nicht nur einen Überschussanteil an elektrischer Energie, sondern erlaubt es zusätzlich zum Alkoholherstellungs- und Entwässerungsprozess auch noch die bei der Destillation anfallende Vinasse aufzukonzentrieren.

Fig. 2 zeigt Einzelheiten der Destillationsanlage 23. Die Anlage ist zweistufig ausgebildet und umfasst eine erste "heißere" Destillationskolonne 59, der über die Leitung 21 und einen Vorwärmer 61 die alkoholhaltige Maische aus der Gärstufe 19 zugeführt wird. Eine zweite "kältere" Destillationskolonne 63 ist der Destillationskolonne 59 maischeeingangsseitig parallel geschaltet und nimmt die alkoholhaltige Maische über einen weiteren Vorerwärmer 65 auf.

Der erste Teil des Abdampfs der Vorkonzentrierungsstufe 15 wird über die Leitung 53 unmittelbar in einen Sumpf 67 der "heißeren" Destillationskolonne 59 zur Beheizung der Destillationskolonne 59 eingespeist. Entsprechend dem Abdampf der Vorkonzentrierungsstufe 15 ist der Druck im Sumpf 67 auf etwa 1,9 bar, a (1,9·10⁵ Pa) und eine Temperatur von 119° C (bei diesem Druck) eingestellt. Es versteht sich, dass der Druck um +/- 0,5 bar (+/- 0,5·10⁵ Pa) schwanken kann. Im Kopfbereich 69 der Destillatonskolonne 59 herrscht dementsprechend ein Druck von 1,6 bar, a (1,6·10⁵ Pa) bei einer Temperatur von etwa 90° C (bei diesem Druck). Auch hier kann der Druck um +/- 0,5 bar (+/- 0,5·10⁵ Pa) abweichen.

Der Destillationskolonne 59 ist ein Kopfbrüdenkondensator 71 zugeordnet, der zugleich einen Reboiler der zweiten Destillationskolonne 63 bildet. Der Kopfbrüdenkondensator 71 kondensiert bei 73 zugeführten alkoholhaltigen Köpfbrüden. Ein Teil des Alkoholkondensats wird bei 75. als Rücklauf in den Kopfbereich der Destillationskolonne 59 zurückgeführt, während ein anderer Teil des Alkoholkondensats der ersten Destillationsstufe 59 bei 77 abgeführt wird. Das Alkoholkondensat kann einer nicht näher dargestellten Rektifikationsstufe zugeführt werden oder aber bei geeigneter rektifizierender Gestaltung der Destillationskolonne 59 über die Leitung 25 direkt der Entwässerungsstufe 27 zugeführt werden.

Die zweite Destillationsstufe 63 arbeitet auf einem niedrigeren Druck- und Temperaturniveau. In ihrem Sumpf 79 herrscht ein Druck von 0,5 bar, a (0,5·10⁵ Pa) bei einer Temperatur von etwa 80° C. Der Druck kann um +/- 0,3 bar (+/- 0,3·10⁵ Pa) schwanken. Die Vinasse der Destillationskolonne 63 zirkuliert, getrieben von einer Pumpe 81 durch den Sumpf 79 und den Kopfbrüdenkondensator 71 zur Kpndensierung des Kopfbrüdens der Destillationskolonne 59. Der Kopfbrüdenkondensator 71 bildet damit zugleich einen Reboiler der Destillationskolonne 63.

Der Destillationskolonne 63 ist gleichfalls ein Kopfbrüdenkondensator 83 zugeordnet, der aus dem Kopfbereich 85 der Destillationskolonne 63 Kopfbrüden bei 87 aufnimmt und bei 89 Alkoholkondensat als Rücklauf der Destillationskolonne 63 wieder zuführt. Ein Teil des Alkoholkondensats wird bei 91 abgezweigt und wiederum entweder über die nicht näher dargestellte Rektifikationsstufe oder bei rektifizierender Gestaltung der Destillationskolonne 63 direkt der Entwässerungsstufe 27 zugeführt. Der Kopfbrüdenkondensator 83 wird aus einer Frischwasserquelle 93 mit Kühlwasser versorgt.

Die Destillationskolonnen 59 und 63 sind alkoholausgangsseitig parallel geschaltet und auch die an Vinasseausgängen 95, 97 der Destillationskolonnen 59, 63 anfallende Vinasse wird in der Verdampferanordnung 33 wieder zusammengeführt.

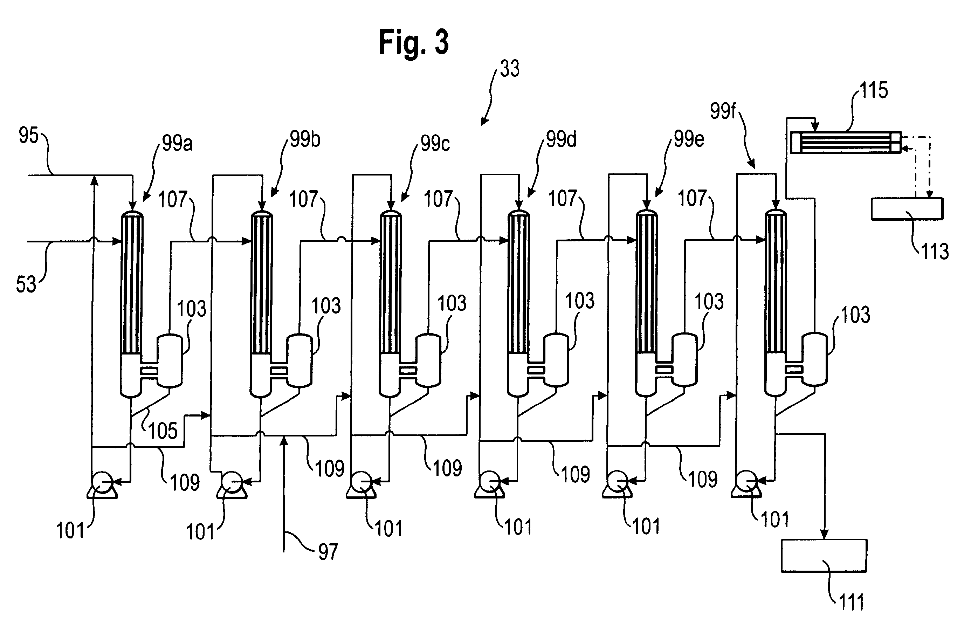

Fig. 3 zeigt Einzelheiten der Verdampferanordnung 33, die die in den Destillationskolonnen 59, 63 der Destillationsanlage 23 anfallende Vinasse auf beispielsweise ein Zehntel ihres Anfangsvolumens aufkonzentriert bzw. eindampft. Die Verdampferanordnung 33 hat im vorliegenden Ausführungsbeispiel sechs Verdampferstufen 99a-99f, die vinassemäßig aber auch energetisch in Reihe geschaltet sind. Die Verdampferstufen 99 sind in diesem Ausführungsbeispiel als Fallfilmverdampfer ausgebildet, was den Vorteil hat, dass die Verdampfung bei relativ kleinen treibenden Temperaturdifferenzen stattfinden kann. Die Temperaturdifferenz zwischen dem treibenden Heizmedium, hier Dampf, und der zu verdampfenden Vinasse beträgt lediglich 3 bis 8° C und liegt typischerweise bei etwa 5° C.

Es versteht sich, dass ein kleinerer Teil der Verdampferstufen auch durch andere Verdampfertypen gebildet sein kann.

Zur besseren Energieausnutzung wird die Vinasse der beiden Destillationskolonnen 59, 63 (Fig. 2) unterschiedlichen Verdampferstufen 99 zugeführt. Die am Ausgang 95 der "heißeren" Destillationskolonne 59 anfallende Vinasse wird dem Kopf der ersten Verdampferstufe 99a zugeführt und zirkuliert über eine Pumpe 101 durch die Verdampferstufe 99a. Beheizt wird die erste Verdampferstufe 99a durch den über die Leitung 53 zugeführten ersten Teil des Abdampfs der Vorkonzentrierungsstufe 15. An den unteren Bereich des Fallfilmverdampfers der ersten Verdampferstufe 99a ist in üblicher Weise ein Abscheider 103 angeschlossen, der den Dampfanteil der Vinassezirkulation vom Konzentratanteil trennt und den Konzentratanteil bei 105 der über die Pumpe 101 geführten Vinassezirkulation wieder zuführt. Der in dem Abscheider 103 abgetrennte Dampfanteil wird bei 107 der nächsten Verdampferstufe 99b als Heizenergie zugeführt. Ein Teil der durch die Verdampferstufe 99a zirkulierenden Vinasse wird ausgangsseitig der Pumpe 101 bei 109 abgezogen und zur weiteren Aufkonzentrierung der nächsten Verdampferstufe 99b zugeführt. Da im vorliegenden Ausführungsbeispiel die Verdampferstufen 99a-99f apparatetechnisch gleich aufgebaut sind, wird zur Beschreibung der Eindampffunktion der einzelnen Verdampferstufen auf die Beschreibung der ersten Verdampferstufe 99a verwiesen. Bei jeder der Verdampferstufen zirkuliert die zu konzentrierende Vinasse durch die Verdampferstufe über die Pumpe 101 und in dem Abscheider 103 jeder Verdampferstufe wird der Abdampf über die Leitung 107 als Heizmedium der nächsten Verdampferstufe zugeführt. Die aufzukonzentrierende Vinasse wird über die Leitung 109 von Stufe zu Stufe aus dem Vinassekreislauf entnommen und dem Vinassekreislauf der nächsten Verdampferstufe zugeführt. Auf diese Weise erhöht sich die Vinassekonzentration von Stufe zu Stufe. Die in der Reihenfolge erste Verdampferstufe 99a ist die "heißeste" Stufe und arbeitet beim höchsten Druck, während die letzte Verdampferstufe 99f die kälteste Verdampferstufe ist und beim niedrigsten Druck arbeitet.

Die über den Vinasseausgang 97 der kälteren zweiten Destillationskolonne 63 abgegebene Vinasse wird ihrem Temperaturniveau entsprechend nicht in die erste Verdampferstufe 99a eingeleitet, sondern im dargestellten Ausführungsbeispiel der dritten Verdampferstufe 99c für die weitere Aufkonzentrierung zugeführt.

Die aufkonzentrierte Vinasse wird aus der letzten Verdampferstufe 99f bei 111 für die weitere Verwendung abgezogen. Der Abdampf aus dem Abscheider 103 der letzten Verdampferstufe 99f wird in einem über eine Kühlwasserquelle 113 gekühlten Kondensator 115 kondensiert.

In dem vorstehend erläuterten Ausführungsbeispiel stimmt die Reihenfolge, mit der die Verdampferstufen 99 vinasseseitig aufeinander folgen mit der Reihenfolge überein, in der die Verdampferstufen 99 Abdampf aus der jeweils vorangegangen Verdampferstufe aufnehmen. Sowohl abdampfseitig als auch vinasseseitig folgen die Verdampferstufen wie folgt aufeinander: 99a, 99b, 99c, 99d, 99e und 99f. Unter Viskositätsgesichtspunkten kann es sinnvoll sein, wenn die vinasseseitige Reihenfolge sich von der abdampfseitigen Reihenfolge unterscheidet. In einer zweckmäßigen Ausgestaltung sind die Verdampferstufen über die Leitungen 107 abdampfseitig nach wie vor in der Reihenfolge 99a, 99b, 99c, 99d, 99e und 99f verbunden, während vinasseseitig die Verdampferstufen über die Leitungen 109 in der Reihenfolge 99a, 99b, 99c, 99f, 99e und 99d miteinander verbunden sind, wobei dementsprechend die Verdampferstufe 99d die aufkonzentrierte Vinasse für die weitere Verarbeitung bei 111 abgibt.

## Patentansprüche

1. Anlage zur Herstellung von Alkohol aus Zuckerrohr, umfassend
- eine das Zuckerrohr zerkleinernde Zerkleinerungsstufe (3),
- eine das zerkleinerte Zuckerrohr in Zuckersaft und Bagasse trennende Extraktionsstufe (7),
- eine den Zuckersaft reinigende Reinigungsstufe (11),
- eine den gereinigten Zuckersaft zu alkoholhaltiger Maische vergärende Gärstufe (19),
- eine die Maische in Rohalkohol und Vinasse trennende Destillationsanlage (23),
- eine den Rohalkohol entwässernde, dehydrierten Alkohol liefernde Entwässerungsstufe (27),
- einen durch Verbrennen der Bagasse beheizten Hochdruckdampferzeuger (39) und
- einen mit dem Hochdruckdampf des Hochdruckdampferzeugers (39) betriebene Dampfturbine (45), die einen elektrischen Strom erzeugenden Generator (47) antreibt,
**dadurch gekennzeichnet, dass**
der gereinigte Zuckersaft der Gärstufe (19) über eine mit Abdampf der Dampfturbine beheizte Vorkonzentrierungsstufe (15) in aufkonzentrierter Form zuführbar ist, und dass die Destillationsanlage (23) sowie die Entwässerungsstufe (27) und eine die Vinasse der Destillationsanlage (23) aufkonzentrierende Verdampferanordnung (33) gemeinsam mit Abdampf der Vorkonzentrierungsstufe beheizt sind,

2. Anlage nach Anspruch 1, **dadurch gekennzeichnet, dass** die Destillationsanlage (23) zweistufig ausgebildet ist und eine mit Abdampf der Vorkonzentrierungsstufe (15) direkt beheizte erste Destillationskolonne (59) sowie eine indirekt durch Kopfbrüden der ersten Destillationskolonne (59) beheizte zweite Destillationskolonne (63) aufweist, die auf einem niedrigeren Kolonnendruck und einer niedrigeren Kolonnentemperatur arbeitet als die erste Destillationskolonne (59).

3. Anlage nach Anspruch 2, **dadurch gekennzeichnet, dass** die beiden Destillationskolonnen (59, 63) maischezuführseitig und gegebenenfalls vinasseausgangsseitig parallel geschaltet sind und dass den beiden Destillationskolonnen (59, 63) gesonderte Kopfbrüdenkondensatoren (71, 83) zugeordnet sind, wobei der Kopfbrüdenkondensator (71) der ersten Destillationskolonne (59) einen Reboiler der zweiten Destillationskolonne (63) bildet.

4. Anlage nach Anspruch 3, **dadurch gekennzeichnet, dass** ein Anteil des Kondensats des Kopfbrüdenkondensators (71, 83) der jeweils zugeordneten Destillationskolonne (59, 63) als Rücklauf zuführbar ist.

5. Anlage nach einem der Ansprüche 2 bis 4, **dadurch gekennzeichnet, dass** die erste Destillationskolonne (59) in ihrem Kopfbereich (69) bei einem Druck von 1,6 +/- 0,5 bar, a (1,6·10⁵ +/- 0,5·10⁵ Pa) und/oder einer Temperatur von etwa 90° C betrieben ist.

6. Anlage nach einem der Ansprüche 2 bis 5, **dadurch gekennzeichnet, dass** die zweite Destillationskolonne (63) in ihrem Sumpfbereich (79) bei einem Druck von 0,5 +/- 0,3 bar, a (0,5·10⁵ +/- 0,3·10⁵ Pa) und/oder einer Temperatur von etwa 80° C betrieben ist.

7. Anlage nach einem der Ansprüche 2 bis 6, **dadurch gekennzeichnet, dass** die Verdampferanordnung (33) mehrere Verdampferstufen (99) aufweist, die jeweils durch Abdampf einer in der Reihe auf einem höheren Druck- oder/und Temperaturniveau vorangehenden Verdampferstufen (99) beheizt sind, und dass eine die Vinasse der ersten Destillationskolonne (59) aufnehmende Verdampferstufe (99a) durch Abdampf der Vorkonzentrierungsstufe (15) direkt beheizt ist

8. Anlage nach Anspruch 7, **dadurch gekennzeichnet, dass** die Vinasse der zweiten Destillationskolonne (63) einer weiteren (99c) der Verdampferstufen (99) zuführbar ist und die Verdampferstufen (99) untereinander vinasseseitig in Reihe geschaltet sind.

9. Anlage nach Anspruch 8, **dadurch gekennzeichnet, dass** die Verdampferanordnung (33) sechs Verdampferstufen (99) aufweist und die Vinasse der zweiten Destillationskolonne (63) der dritten Verdampferstufe (99c) zuführbar ist.

10. Anlage nach einem der Ansprüche 7 bis 9, **dadurch gekennzeichnet, dass** die Verdampferstufen (99) vinasseseitig in einer Reihenfolge in Reihe geschaltet sind, die von der Reihenfolge mit der die Verdampferstufen abdampfseitig aufeinander folgen, verschieden ist.

11. Anlage nach Anspruch 10, **dadurch gekennzeichnet, dass** die Verdampferanordnung (33) sechs Verdampferstufen (99) aufweist und dass bezogen auf die Abdampfreihenfolge in der vinasseseitigen Reihenfolge die zweite Verdampferstufe auf die erste Verdampferstufe, die dritte auf die zweite, die sechste auf die dritte, die fünfte auf die sechste und die vierte Verdampferstufe auf die fünfte Verdampferstufe folgt.

12. Anlage nach einem der Ansprüche 7 bis 11, **dadurch gekennzeichnet, dass** die Verdampferstufen (99) als Fallfilmverdampfer ausgebildet sind.

13. Anlage nach einem der Ansprüche 1 bis 12, **dadurch gekennzeichnet, dass** die Verdampferanordnung (33) mehrere vinasseseitig in Reihe geschaltete Fallfilmverdampferstufen aufweist, von denen zumindest die in der Reihe erste Fallfilmverdampferstufe (99a) durch Abdampf der Vorkonzentrierungsstufe (15) beheizt ist.

14. Anlage nach einem der Ansprüche 1 bis 13, **dadurch gekennzeichnet, dass** die Vorkonzentrierungsstufe (15) Abdampf mit einem Druck von 2 +/- 0,5 bar, a (2·10⁵ +/- 0,5·10⁵ Pa) und/oder einer Temperatur von etwa 120° C erzeugt.

15. Anlage nach einem der Ansprüche 1 bis 14, **dadurch gekennzeichnet, dass** die Vorkonzentrierungsstufe (15) als Fallfilmverdampfer oder als Plattenverdampfer ausgebildet ist.

## Claims

1. System for producing alcohol from sugar cane, comprising
- a comminution stage (3) where the sugar cane is comminuted,
- an extraction stage (7) separating the comminuted sugar cane into sugar cane juice and bagasse,
- a purification stage (11) purifying the sugar cane juice,
- a fermentation stage (19) fermenting the purified sugar cane juice to form an alcohol-containing mash,
- a distillation system (23) separating the mash into raw alcohol and vinasse,
- a dewatering stage (27) dewatering the raw alcohol and supplying dehydrated alcohol, ,
- a high pressure boiler (39) heated by burning the bagasse and
- a steam turbine (45), which is operated by the high pressure steam of the high pressure boiler (39) and drives a generator (47) generating an electric current,
**characterised in that** the purified sugar cane juice of the fermentation stage (19) can be fed in concentrated form by means of a preconcentration stage (15) heated with exhaust steam of the steam turbine, and **in that** the distillation system (23) as well as the dewatering stage (27) and an evaporator arrangement (33) concentrating the vinasse of the distillation system (23) are heated together with exhaust steam of the preconcentration stage.

2. System according to claim 1, **characterised in that** the distillation system (23) is configured in two stages and has a first distillation column (59) directly heated with exhaust steam of the preconcentration stage (15) and a second distillation column (63), which is heated indirectly by head exhaust vapours of the first distillation column (59) and works to a lower column pressure and a lower column temperature than the first distillation column (59).

3. System according to claim 2, **characterised in that** the two distillation columns (59, 63) are connected in parallel on the mash feed side and optionally on the vinasse outlet side and **in that** separate head exhaust vapour condensers (71, 83) are assigned to the two distillation columns (59, 63), the head exhaust vapour condenser (71) of the first distillation column (59) forming a reboiler of the second distillation column (63).

4. System according to claim 3, **characterised in that** a proportion of the condensate of the head exhaust vapour condenser (71, 83) can be fed as a return flow to the respectively assigned distillation column (59, 63).

5. System according to any one of claims 2 to 4, **characterised in that** the first distillation column (59) in its head region (69) is operated at a pressure of 1.6 +/- 0.5 bar, a (1.6 · 10⁵ +/- 0.5 · 10⁵ Pa) and/or a temperature of about 90°C.

6. System according to any one of claims 2 to 5, **characterised in that** the second distillation column (63) in its sump region (79) is operated at a pressure of 0.5 +/- 0.3 bar, a (0.5 · 10⁵ +/- 0.3 · 10⁵ Pa) and/or a temperature of about 80°C.

7. System according to any one of claims 2 to 6, **characterised in that** the evaporator arrangement (33) has a plurality of evaporator stages (99), which are in each case heated by the exhaust steam of a preceding evaporator stage (99) in the series to a higher pressure or/and temperature level, and **in that** an evaporator stage (99a) receiving the vinasse of the first distillation column (59) is directly heated by exhaust steam of the preconcentration stage (15).

8. System according to claim 7, **characterised in that** the vinasse of the second distillation column (63) can be fed to a further one (99c) of the evaporation stages (99) and the evaporator stages (99) are connected in series to one another on the vinasse side.

9. System according to claim 8, **characterised in that** the evaporator arrangement (33) has six evaporator stages (99) and the vinasse of the second distillation column (63) can be fed to the third evaporator stage (99c).

10. System according to any one of claims 7 to 9, **characterised in that** the evaporator stages (99) are connected in series on the vinasse side in an order, which is different from the order in which the evaporator stages follow one another on the exhaust steam side.

11. System according to claim 10, **characterised in that** the evaporator arrangement (33) has six evaporator stages (99) and **in that**, in relation to the exhaust steam order in the order on the vinasse side, the second evaporator stage follows the first evaporator stage, the third follows the second, the sixth follows the third, the fifth follows the sixth and the fourth evaporator stage follows the fifth evaporator stage.

12. System according to any one of claims 7 to 11, **characterised in that** the evaporator stages (99) are configured as falling film evaporators.

13. System according to any one of claims 1 to 12, **characterised in that** the evaporator arrangement (33) has a plurality of falling film evaporator stages connected in series on the vinasse side, of which at least the first falling film evaporator stage (99a) in the series is heated by exhaust steam of the preconcentration stage (15).

14. System according to any one of claims 1 to 13, **characterised in that** the preconcentration stage (15) generates exhaust steam at a pressure of 2 +/- 0.5 bar, a (2 · 10⁵ +/- 0.5 · 10⁵ Pa) and/or a temperature of about 120°C.

15. System according to any one of claims 1 to 14, **characterised in that** the preconcentration stage (15) is configured as a falling film evaporator or as a plate evaporator.

## Revendications

1. Installation de production d'alcool à partir de canne à sucre, comprenant
- un étage de broyage (3) broyant la canne à sucre,
- un étage d'extraction (7) séparant la canne à sucre broyée en vesou et en bagasse,
- un étage de purification (11) purifiant le vesou,
- un étage de fermentation (19) faisant fermenter le vesou purifié en moût alcoolisé,
- une installation de distillation (23) séparant le moût en alcool brut et en vinasse,
- un étage de déshydratation (27) déshydratant l'alcool brut et fournissant un alcool déshydraté,
- un générateur de vapeur à haute pression (39) chauffé par la combustion de la vinasse, et
- une turbine à vapeur (45) entraînée par la vapeur à haute pression du générateur de vapeur à haute pression (39), qui entraîne un générateur (47) produisant du courant électrique,
**caractérisée en ce que** le vesou purifié de l'étage de fermentation (19) peut être amené à une forme concentrée au moyen d'un étage de préconcentration (15) chauffé avec la vapeur résiduelle de la turbine à vapeur, et **en ce que** l'installation de distillation (23) ainsi que l'étage de déshydratation (27) et un dispositif d'évaporateur (33) concentrant la vinasse de l'installation de distillation (23) sont chauffés en commun avec la vapeur résiduelle de l'étage de préconcentration.

2. Installation selon la revendication 1, **caractérisée en ce que** l'installation de distillation (23) est réalisée à deux étages et présente une première colonne de distillation (59) chauffée directement avec la vapeur résiduelle de l'étage de préconcentration (15) ainsi qu'une deuxième colonne de distillation (63) chauffée indirectement par la vapeur chaude de tête de la première colonne de distillation (59), qui fonctionne à une pression de colonne plus basse et une température de colonne plus basse que la première colonne de distillation (59).

3. Installation selon la revendication 2, **caractérisée en ce que** les deux colonnes de distillation (59, 63) sont connectées en parallèle du côté de l'arrivée de moût et éventuellement du côté de la sortie de vinasse et **en ce que** des condenseurs de vapeur chaude de tête distincts (71, 83) sont associés aux deux colonnes de distillation (59, 63), dans laquelle le condenseur de vapeur chaude de tête (71) de la première colonne de distillation (59) forme un rebouilleur de la deuxième colonne de distillation (63).

4. Installation selon la revendication 3, **caractérisée en ce qu'**une part du condensat du condenseur de vapeur chaude de tête (71, 83) de la colonne de distillation respectivement associée (59, 63) peut être fournie sous forme de retour.

5. Installation selon l'une quelconque des revendications 2 à 4, **caractérisée en ce que** la première colonne de distillation (59) est conduite dans sa région de tête (69) à une pression de 1,6±0,5 bar, a (1,6.10⁵±0,5.10⁵ Pa) et/ou à une température d'environ 90°C.

6. Installation selon l'une quelconque des revendications 2 à 5, **caractérisée en ce que** la deuxième colonne de distillation (63) est conduite dans sa région de fond (79) à une pression de 0,5±0,3 bar,a (0,5.10⁵±0,3.10⁵ Pa) et/ou à une température d'environ 80°C.

7. Installation selon l'une quelconque des revendications 2 à 6, **caractérisée en ce que** le dispositif d'évaporateur (33) comporte plusieurs étages d'évaporateur (99), qui sont respectivement chauffés par la vapeur résiduelle d'un des étages d'évaporateur (99) précédents dans la série à un niveau de pression et/ou un niveau de température plus élevé, et **en ce qu'**un étage d'évaporateur (99a) recevant la vinasse de la première colonne de distillation (59) est chauffé directement par la vapeur résiduelle de l'étage de préconcentration (15).

8. Installation selon la revendication 7, **caractérisée en ce que** la vinasse de la deuxième colonne de distillation (63) peut être fournie à un autre (99c) des étages d'évaporateur (99) et les étages d'évaporateur (99) sont connectés en série l'un à l'autre du côté de la vinasse.

9. Installation selon la revendication 8, **caractérisée en ce que** le dispositif d'évaporateur (33) comporte six étages d'évaporateur (99) et la vinasse de la deuxième colonne de distillation (63) peut être fournie au troisième étage d'évaporateur (99c).

10. Installation selon l'une quelconque des revendications 7 à 9, **caractérisée en ce que** les étages d'évaporateur (99) sont connectés du côté de la vinasse en série avec un ordre, qui est différent de l'ordre avec lequel les étages d'évaporateur se suivent du côté de la vapeur résiduelle.

11. Installation selon la revendication 10, **caractérisée en ce que** le dispositif d'évaporateur (33) comporte six étages d'évaporateur (99) et **en ce que**, par rapport à l'ordre de la vapeur résiduelle dans la série du côté de la vinasse, le deuxième étage d'évaporateur suit le premier étage d'évaporateur, le troisième suit le deuxième, le sixième suit le troisième, le cinquième suit le sixième et le quatrième étage d'évaporateur suit le cinquième étage d'évaporateur.

12. Installation selon l'une quelconque des revendications 7 à 11, **caractérisée en ce que** les étages d'évaporateur (99) sont formés par des évaporateurs à film tombant.

13. Installation selon l'une quelconque des revendications 1 à 12, **caractérisée en ce que** le dispositif d'évaporateur (33) comporte plusieurs étages d'évaporateur à film tombant connectés en série du côté de la vinasse, dont au moins le premier étage d'évaporateur à film tombant (99a) dans la série est chauffé par la vapeur résiduelle de l'étage de préconcentration (15).

14. Installation selon l'une quelconque des revendications 1 à 13, **caractérisée en ce que** l'étage de préconcentration (15) produit de la vapeur résiduelle avec une pression de 2±0,5 bar,a (2.10⁵±0,5.10⁵ Pa) et/ou une température d'environ 120°C.

15. Installation selon l'une quelconque des revendications 1 à 14, **caractérisée en ce que** l'étage de préconcentration (15) est formé par un évaporateur à film tombant ou par un évaporateur à plaques.
